# EUROPÄISCHE PATENTANMELDUNG

(11) **EP 1 352 607 A1**
(43) Veröffentlichungstag der Anmeldung: **15.10.2003**
(21) Anmeldenummer: 02008043.8
(22) Anmeldetag: 10.04.2002
(51) Int. Cl.: A61B 5/00, G06F 19/00

(54) **Verfahren und System zur Überwachung des Therapieverlaufs einer medizinischen Behandlung**

(71) Anmelder: SIEMENS AKTIENGESELLSCHAFT, 80333 München (DE); Dr. Hein GmbH, 90429 Nürnberg (DE)
(72) Erfinder: Abraham-Fuchs, Klaus, 91058 Erlangen (DE); Eisermann, Uwe, 91052 Erlangen (DE); Hein, Achim, 90403 Nürnberg (DE); Richter, Nils, 95349 Thurnau (DE); Setz, Robert, 91126 Rednitzhembach (DE)
(74) Vertreter: Berg, Peter, Dipl.-Ing.

(57) **Zusammenfassung**

Die vorliegende Erfindung betrifft ein Verfahren sowie ein System zur Überwachung des Therapieverlaufs einer medizinischen Behandlung eines Patienten, bei dem ein erstes Zustandsprofil des Patienten durch Messung eines oder mehrerer zu therapierender Defizite zu Beginn der Behandlung und in vorgebbaren Zeitabständen während der Behandlung erstellt wird. Eine aus dem gemessenen Zustandsprofil abgeleitete Maßzahl für den Therapiefortschritt wird vorzugsweise automatisch mit einer Vergleichs-Kurve verglichen, die aus den Therapie-Verlaufsdaten einer Vielzahl von Vergleichspatienten gebildet wird, die eine vergleichbare Therapie bereits durchgeführt haben. Das vorliegende Verfahren sowie das zugehörige System ermöglichen es dem Arzt oder Therapeuten, automatisiert und ohne Zeitaufwand den Therapiefortschritt eines Patienten auch bei Durchführung einer telemedizinischen Behandlung jederzeit erkennen zu können.

## Beschreibung

Die vorliegende Erfindung betrifft ein Verfahren sowie ein System zur Überwachung des Therapieverlaufs einer medizinischen Behandlung eines Patienten, wie sie insbesondere im Rahmen einer Rehabilitation oder im Zusammenhang mit Disease Management Services für Patienten mit chronischen Krankheiten durchgeführt wird. Ein besonderes Anwendungsfeld stellen telemedizinische Behandlungsformen dar, bei denen der Patient zur Therapie gehörende Trainingseinheiten in seinem häuslichen Umfeld durchführt und lediglich über eine Datenverbindung mit dem betreuenden Arzt oder Therapeuten in Verbindung steht. Die Erfindung betrifft weiterhin ein Verfahren zur Bereitstellung von Vergleichsdaten für die Überwachung des Fortschritts oder die Bewertung des Erfolges einer derartigen Therapie.

Therapiemaßnahmen in der Rehabilitation zielen vorrangig auf die Wiedererlangung körperlicher Fähigkeiten ab, die krankheits- oder unfallbedingt ganz oder teilweise abhanden gekommen sind. Zu diesem Zweck werden dem Patienten Übungsprogramme verordnet, mit deren Hilfe er diese Fähigkeiten trainieren kann, um die diesbezüglichen Defizite gegenüber einem Normalzustand zu verringern oder auszugleichen. Der Prozess der Regeneration von Fähigkeiten durch derartige Trainingsprogramme dauert jedoch häufig sehr lange, so dass ein stationärer Aufenthalt in einem Krankenhaus oder einer Klinik über diesen gesamten Zeitraum bereits aus Kostengründen nicht möglich ist. Der Patient wird daher in der Regel die Trainingsmaßnahmen nach seiner Entlassung aus der Klinik im häuslichen Umfeld fortführen, in dem nur eine sporadische Betreuung durch medizinisches Fachpersonal möglich ist. Um diese Situation zu verbessern, werden zunehmend telemedizinische Behandlungsformen eingesetzt, bei welchen der Patient durch eine digitale Datenverbindung mit medizinischem Fachpersonal in Verbindung steht, so dass eine weiterführende Betreuung des Patienten auch während des Therapieprozesses im häuslichen Umfeld ermöglicht wird.

Ein Problem dieser neuen Art der telemedizinischen Behandlung besteht darin, dass der behandelnde Arzt oder Therapeut den Patienten nur noch in größeren Zeitabständen persönlich sieht, so dass die Beurteilung des Therapiefortschrittes bezogen auf die individuelle Situation des Patienten erschwert wird. Diese Problematik stellte sich in der Vergangenheit nicht, da bei den bisher eingesetzten herkömmlichen Behandlungsformen der Arzt oder Therapeut in ausreichendem Ausmaß persönlichen Kontakt zum Patienten hatte. Bei diesem persönlichen Kontakt reicht im Allgemeinen die Erfahrung des Arztes oder Therapeuten aus, um im Gespräch und mittels visueller Beurteilung die Therapiefortschritte des Patienten beurteilen und einordnen zu können. Der persönliche Kontakt mit dem Patienten war für die Beurteilung und Einordnung des Therapiefortschrittes bisher schon deswegen unumgänglich, da das Ausmaß bestehender Fähigkeitsdefizite und das Potential des Patienten zur Wiederherstellung der Fähigkeiten individuell sehr unterschiedlich und somit nicht einfach durch ein standardisiertes Verfahren erfassbar ist.

Die Aufgabe der vorliegenden Erfindung besteht darin, ein Verfahren sowie ein System zur Überwachung des Therapieverlaufs einer medizinischen Behandlung eines Patienten anzugeben, mit denen automatisiert und ohne Zeitaufwand für den Arzt oder Therapeuten der Therapiefortschritt des Patienten beurteilt werden kann. Das Verfahren sowie das System sollen sich insbesondere für den Einsatz bei telemedizinischen Behandlungsformen eignen, bei denen der Arzt oder Therapeut nur noch in geringem Maße direkten Kontakt mit dem Patienten hat.

Die Aufgabe wird mit dem Verfahren sowie dem System der Patentansprüche 1 bzw. 21 gelöst. Vorteilhafte Ausgestaltungen des Verfahrens sowie des Systems sind Gegenstand der Unteransprüche oder lassen sich aus der nachfolgenden Beschreibung und den Ausführungsbeispielen entnehmen. Die Patentansprüche 19 und 20 geben weiterhin ein Verfahren zur Bereitstellung von Vergleichsdaten für die Überwachung des Fortschritts oder die Bewertung des Erfolges einer Therapie an, das bei dem vorliegenden Verfahren zur Überwachung des Therapieverlaufs zum Einsatz kommen kann.

Bei dem vorliegenden Verfahren zur Überwachung des Therapieverlaufs einer medizinischen Behandlung eines Patienten wird zunächst ein erstes Zustandsprofil des Patienten durch Messung eines oder mehrerer zu therapierender Defizite zu Beginn der Therapie erstellt, aus denen mit einer Berechnungsvorschrift eine für den Therapiefortschritt repräsentative Maßzahl berechnet wird. Der Begriff Defizit ist hierbei im weiteren Sinne zu verstehen, so dass er nicht nur Fähigkeitsoder Fertigungsdefizite umfasst, sondern jede Abweichung einer messbaren Eigenschaft von einem vorgebbaren Behandlungsziel der Therapie. Das Zustandsprofil entspricht dabei dem momentanen Status der jeweiligen messbaren Eigenschaften, aus denen mit einer geeigneten Berechnungsvorschrift eine Maßzahl für den momentanen Zustand des Patienten berechnet wird, die der obigen repräsentativen Maßzahl für den Therapiefortschritt entspricht. Für das Verfahren werden Therapieverlaufsdaten einer Vielzahl von Vergleichs-Patienten eines Patienten-Kollektivs bereitgestellt, die eine vergleichbare Therapie bereits durchgeführt haben. Vorzugsweise wird aus diesen Vergleichs-Patienten ein Sub-Kollektiv ausgewählt, das nur Vergleichs-Patienten mit einem innerhalb vorgebbarer Grenzwerte mit dem ersten Zustandsprofil des zu therapierenden Patienten übereinstimmenden Zustandsprofil zu Beginn der Therapie umfasst. Aus den Therapieverlaufsdaten der Vergleichs-Patienten des Patienten-Kollektivs oder des Sub-Kollektivs wird mit Hilfe der Berechnungsvorschrift für die repräsentative Maßzahl eine Therapie-Verlaufskurve gebildet und abgespeichert, die als Vergleichskurve für die durchzuführende Therapie des Patienten dient. Im Verlauf der Therapie des vorliegenden Patienten werden nun zu unterschiedlichen Zeitpunkten bzw. in unterschiedlichen Therapiestufen weitere Zustandsprofile des Patienten durch Messung der zu therapierenden Defizite erstellt und aus den Messwerten jeweils mit Hilfe der Berechnungsvorschrift die aktuelle Maßzahl berechnet, die ein Maß für den aktuellen Therapiefortschritt darstellt. Die jeweils auf diese Weise erfasste aktuelle Maßzahl des Patienten wird mit der dem jeweiligen Zeitpunkt bzw. der jeweiligen Therapiestufe entsprechenden Maßzahl der abgespeicherten Vergleichskurve durch eine erste Datenverarbeitungsstation in geeigneter Weise, insbesondere graphisch, an einem Anzeigegerät dargestellt und/oder automatisch verglichen und das Vergleichsergebnis dargestellt. Auf Basis dieser Darstellung kann der Arzt oder Therapeut sofort den aktuellen Therapiefortschritt des Patienten erkennen. Die Zeitabstände, in denen eine jeweils neue Maßzahl berechnet wird, können frei gewählt werden und hängen von der Art der Therapie ab.

Vorzugsweise wird die Messung der zu therapierenden Defizite im Verlauf der Therapie automatisch durch eine zweite Datenverarbeitungsstation vorgenommen, die die gemessenen Werte und/oder die daraus berechnete aktuelle Maßzahl über ein Netzwerk an die erste Datenverarbeitungsstation übermittelt. Durch diese Ausgestaltung wird gerade der Einsatz einer telemedizinischen Behandlung ermöglicht, bei der die zweite Datenverarbeitungsstation im häuslichen Umfeld des Patienten steht, während die erste Datenverarbeitungsstation dem Arzt oder Therapeuten in seiner Praxis zur Verfügung steht. Für die Messung der zu therapierenden Defizite ist die zweite Datenverarbeitungsstation gegebenenfalls mit entsprechenden Schnittstellen ausgestattet und mit Messwertaufnehmern zur Erfassung der Messwerte verbunden. Im Falle kognitiver Fähigkeitsdefizite kann die Messwertaufnahme selbstverständlich auch direkt an der zweiten Datenverarbeitungsstation erfolgen, indem diese dem Patienten entsprechende am Bildschirm zu lösende Aufgaben oder einen Fragebogen bereitstellt, und das Ergebnis entsprechend auswertet.

In einer weiteren sehr vorteilhaften Ausführungsform des vorliegenden Verfahrens wird zusätzlich eine zulässige Abweichung der im Therapieverlauf ermittelten Maßzahlen des Therapiefortschritts des Patienten von der Vergleichskurve festgelegt und in geeigneter Weise dargestellt, wobei die erste und/oder zweite Datenverarbeitungsstation bei Feststellen einer Überschreitung dieser Abweichung auch eine Warnmeldung erzeugen können. Diese Warnmeldung wird dem Arzt oder Therapeuten direkt zur Kenntnis gebracht, beispielsweise durch eine entsprechende Meldung am Monitor der ersten Datenverarbeitungsstation oder durch eine entsprechende E-Mail an den Arzt oder Therapeuten. Auf diese Weise wird der Arzt oder Therapeut benachrichtigt, wenn die Entwicklung des Patienten aus einem erwarteten Bereich herausfällt, so dass gegebenenfalls entsprechende Gegen- oder Folge-Maßnahmen ergriffen werden können.

Bei dem vorliegenden Verfahren wird ausgenutzt, dass im Zeitalter elektronischer Patientenakten und digitaler Krankenhaus-Informationssysteme die Therapiemaßnahmen und der Therapieverlauf für alle behandelten Patienten aufgezeichnet wird. Damit stehen künftig Patientendaten von sehr großen Patientenkollektiven zur automatisierten Auswertung zur Verfügung, die trotz der großen individuellen Unterschiede im Krankheitsbild des einzelnen Patienten einen statistisch signifikanten Vergleich eines individuellen Patienten gegen das Therapieergebnis bzw. den Therapieverlauf im Durchschnitt eines großen Patientenkollektivs erlauben. Während ein Vergleich zwischen zwei Einzelpatienten wenig Aussagekraft besitzt, können in großen Patientenkollektiven die Erfahrungen aus vielen Krankheitsgeschichten zu Mittelwerten zusammengefasst werden, die nunmehr eine Aussagekraft für den Vergleich mit einem individuellen Patienten im Sinne eines Benchmark besitzen.

Mit dem vorliegenden Verfahren und dem zugehörigen System, die diesen Vergleich eines individuellen Patienten zu verschiedenen Zeitpunkten der Therapie mittels einer geeigneten Maßzahl mit einem repräsentativen historischen Patientenkollektiv nutzen, lässt sich für den zuständigen Arzt oder Therapeuten anhand der automatisiert erstellten Vergleichsdarstellung jederzeit der Therapiefortschritt des Patienten erkennen und einordnen. Ein direkter persönlicher Kontakt zu dem Patienten ist hierfür nicht erforderlich. Das Verfahren und das System ermöglichen somit eine Fernüberwachung des Therapiefortschritts eines Patienten, beispielsweise bei rehabilitativen Trainingsprogrammen, die mit sehr geringem Zeitaufwand für den Arzt oder Therapeuten verbunden ist. Das Verfahren lässt sich nach Beginn der Therapie, d.h. nach der Festlegung der Therapiemaßnahmen und dem Erstellen des ersten Zustandsprofils des Patienten, auch vollständig automatisiert weiterführen, so dass der Arzt oder Therapeut automatisch in gewissen Zeitabständen über den Therapiefortschritt informiert oder gegebenenfalls bei signifikanten Abweichungen von einem erwarteten Therapieverlauf automatisch informiert wird.

Für die Bereitstellung von Vergleichsdaten bzw. einer Vergleichskurve beim vorliegenden Verfahren kann ein Verfahren zur Bereitstellung von Vergleichsdaten eingesetzt werden, das sich auch für andere Anwendungen eignet. Bei diesem Verfahren sind die Therapieverlaufsdaten einer Vielzahl von Patienten eines Patienten-Kollektivs in einer ersten Datenbank abgelegt. Aus einer zweiten Datenbank wird eine Berechnungsvorschrift für die Berechnung einer Maßzahl zur Bewertung des Therapiefortschrittes oder eines Therapieerfolges sowie von Auswahlparametern für eine Auswahl eines repräsentativen Sub-Kollektivs aus der Vielzahl von Vergleichs-Patienten abgerufen. Diese zweite Datenbank enthält für eine Vielzahl von Ausgangs-Zustandsprofilen die entsprechenden Parameter für eine repräsentative Auswahl eines Sub-Kollektivs sowie Berechnungsvorschriften für Maßzahlen zur Bewertung des Therapiefortschrittes oder Therapieerfolges. Nach Abrufen der Berechnungsvorschrift sowie der Parameter für die Auswahl des Sub-Kollektivs erfolgt diese Auswahl automatisch durch die erste Datenverarbeitungsstation mit den aus der zweiten Datenbank erhaltenen Parametern. Nach dieser automatischen Auswahl des geeigneten Sub-Kollektivs werden mit der ebenfalls abgerufenen Berechnungsvorschrift durch die erste Datenverarbeitungsstation ein oder mehrere Maßzahlen für die Bewertung des Therapiefortschrittes oder des Therapieerfolges aus den Therapieverlaufsdaten des Sub-Kollektivs berechnet. Durch Darstellung der entsprechenden Maßzahlen in einer Therapie-Verlaufskurve wird die Vergleichskurve erhalten.

Dieses Verfahren zur Bereitstellung von Vergleichsdaten für die Überwachung des Fortschritts oder die Bewertung des Erfolges einer Therapie lässt sich vorteilhaft auch für den Vergleich der Therapieerfolge unterschiedlicher medizinischer Leistungserbringer einsetzen. Hierfür werden die Patienten-Kollektive der jeweiligen Leistungserbringer durch Bilden der entsprechenden Sub-Kollektive und Berechnen einer aussagekräftigen Maßzahl direkt miteinander verglichen. Dieses Verfahren ermöglicht somit zum einen ein Benchmarking der Qualität von Rehabilitations-Institutionen oder Disease Management Services und stellt zum anderen eine repräsentative Größe für den Therapieerfolg aus den Therapieverlaufsdaten eines historischen Patienten-Kollektivs zur Verfügung.

Das vorliegende System zur Überwachung des Therapieverlaufs einer medizinischen Behandlung eines Patienten umfasst zumindest eine erste Datenverarbeitungsstation sowie eine zweite Datenverarbeitungsstation, die zumindest zeitweise über ein Netzwerk miteinander Daten austauschen können. Die erste Datenverarbeitungsstation ist mit einer ersten Datenbank verbunden, die Therapieverlaufsdaten einer Vielzahl von Vergleichs-Patienten eines Patienten-Kollektivs enthält, die eine vergleichbare Therapie bereits durchgeführt haben. Die erste Datenverarbeitungsstation umfasst weiterhin ein Modul zur Bildung und Speicherung einer Therapie-Verlaufskurve aus den Therapieverlaufsdaten des Patienten-Kollektivs mit Hilfe einer Berechnungsvorschrift für eine den Therapieerfolg repräsentierenden Maßzahl, zur Berechnung der jeweils aktuellen Maßzahl des Patienten aus den übermittelten Messwerten sowie zur Darstellung der jeweils aktuellen Maßzahl zusammen mit der dem jeweiligen Zeitpunkt bzw. der jeweiligen Therapiestufe entsprechenden Maßzahl der abgespeicherten Vergleichskurve und/oder zum automatischen Vergleich der jeweils aktuellen Maßzahl mit der Therapie-Vergleichskurve und zur Ausgabe des Vergleichsergebnisses. Die zweite Datenverarbeitungseinrichtung umfasst ein Modul zur Erfassung der Messwerte für die zu therapierenden Defizite und zur Übertragung dieser Messwerte an die erste Datenverarbeitungsstation.

Vorzugsweise ist die erste Datenverarbeitungsstation mit einer zweiten Datenbank verbunden, in der für eine Vielzahl von Zustandsprofilen Parameter für eine repräsentative Auswahl eines Sub-Kollektivs aus dem Patienten-Kollektiv der Vergleichs-Patienten sowie die Berechnungsvorschrift für die repräsentative Maßzahl angegeben sind. Das erste Modul ist in dieser Ausführungsform zur Auswahl des Sub-Kollektivs aus dem Patienten-Kollektiv auf Basis der von der zweiten Datenbank erhaltenen Parameter und zur Bildung der Therapie-Verlaufskurve aus den Therapieverlaufsdaten des Sub-Kollektivs ausgebildet.

Mit dem vorliegenden Verfahren zur Überwachung des Therapieverlaufs sowie dem zugehörigen System lässt sich automatisiert und ohne Zeitaufwand der Therapiefortschritt von Patienten bei Rehabilitationsmaßnahmen oder Disease Management Programmen überwachen. Der Therapieverlauf wird hierbei durch geeignete Kennzahlen gemessen und gegen den in gleicher Art gemessenen und vorzugsweise gemittelten Therapieverlauf eines historischen Patienten-Kollektivs verglichen. In weiteren Ausprägungen des Verfahrens können Warnhinweise und Alarme aus dem Vergleich abgeleitet und dem Arzt zur Kenntnis gebracht werden.

Das vorliegende Verfahren zur Überwachung des Therapieverlaufs sowie das zugehörige System werden nachfolgend anhand von Ausführungsbeispielen in Verbindung mit den Zeichnungen nochmals erläutert. Hierbei zeigen:
- Fig. 1: einen schematischen Überblick über einen möglichen Verfahrensablauf des vorliegenden Verfahrens;
- Fig. 2: ein Beispiel für ein erstes Zustandsprofil des Patienten;
- Fig. 3: ein Beispiel für eine Vergleichskurve mit zulässigen Ober- und Untergrenzen, bei der der Therapieverlauf des Patienten innerhalb der zulässigen Grenzwerte liegt;
- Fig. 4: ein Beispiel für die gleiche Vergleichskurve, bei der der Therapieverlauf des Patienten die zulässigen Grenzen überschreitet;
- Fig. 5: ein weiteres Beispiel für eine Therapie-Verlaufskurve mit zulässigen Ober- und Untergrenzen, bei der der Therapieverlauf des Patienten die zulässigen Grenzwerte überschreitet;
- Fig. 6: ein Beispiel für eine mögliche Darstellung des Therapiefortschritts in Bezug auf einzelne zu therapierende Defizite;
- Fig. 7: ein weiteres Beispiel für eine mögliche Darstellung des Therapiefortschritts in Bezug auf einzelne zu therapierende Defizite sowie der zugehörige Grad der Compliance des Patienten; und
- Fig. 8: schematisch ein Beispiel für den Aufbau des vorliegenden Systems zur Durchführung des Verfahrens.

Im Folgenden werden beispielhaft einzelne Verfahrensschritte zur Überwachung des Therapieverlaufs bei einem Rehabilitationstraining eines Patienten dargestellt. Zu Beginn des Verfahrens wird das Zustandsprofil des Patienten durch Messung eines oder mehrerer zu therapierender Defizite gemessen. Dieses Zustandsprofil ist im vorliegenden Beispiel ein Fähigkeitsprofil, in dem für jede für die Therapie maßgebliche Fähigkeit das Ausmaß des Defizits durch eine Messung quantifiziert, beispielsweise in Prozent der Fähigkeit einer durchschnittlichen gesunden Person angegeben und in einer Datenbank 1 abgespeichert wird.

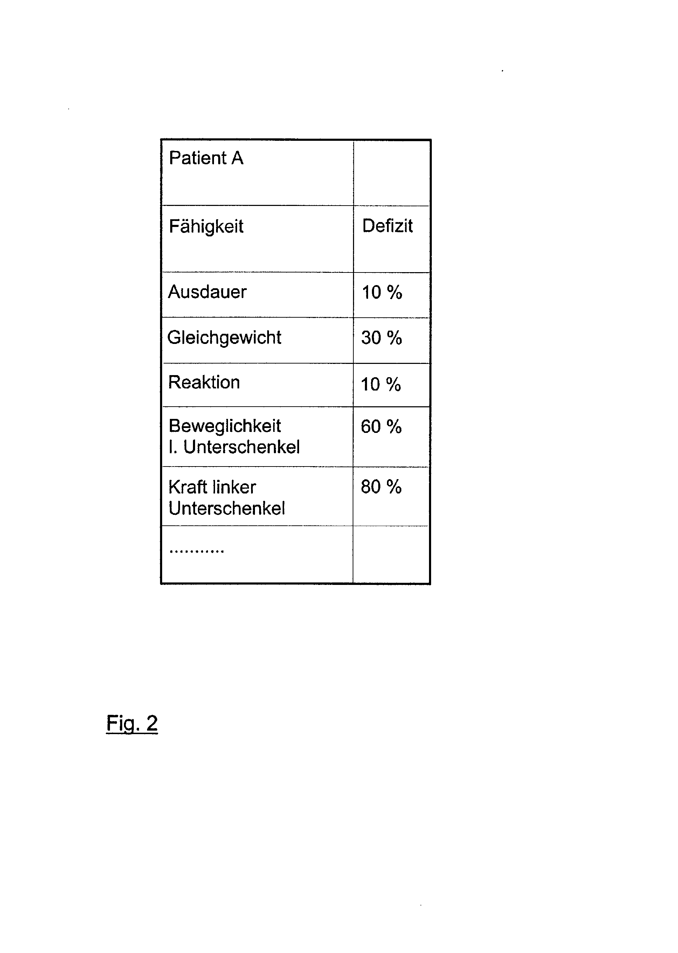

Fig. 2 zeigt ein Beispiel für ein derartiges individuelles Fähigkeitsprofil eines Patienten A in tabellarischer Form. Dieses Fähigkeitsprofil enthält als zu therapierende Defizite die Fähigkeiten Ausdauer, Gleichgewicht, Reaktion, Beweglichkeit des linken Unterschenkels sowie Kraft des linken Unterschenkels. Selbstverständlich kann ein derartiges Fähigkeitsprofil auch weitere Defizite zu therapierender Fähigkeiten enthalten, wie dies mit den Punkten in der Figur angedeutet ist. Das jeweilige Defizit in Prozent der Fähigkeit einer normalen gesunden Vergleichsperson ist im rechten Teil der Tabelle dargestellt. Die Messung dieses Fähigkeitsprofils zu Beginn der Therapie kann in üblicher Weise durch dem Fachmann bekannte Maßnahmen, beispielsweise für die Reaktion durch ein entsprechendes Computer-Programm oder für die Ausdauer durch eine Ergometer-Messung, erfasst werden. Die Messung dieses Zustandsprofils wird im Verlauf der Therapie in gewissen Zeitabständen wiederholt. Das Ende der Therapie ist erreicht, wenn das Fähigkeitsprofil entsprechend dem der gesunden Vergleichsperson in ausreichendem Maß wieder hergestellt ist. Die Messung eines ausreichend wieder hergestellten Fähigkeitsprofils des Patienten kann hierbei der Anlass für die Entscheidung sein, die Therapie zu beenden.

Zu Beginn der Therapie werden weiterhin durch eine Datenbank 2 eine Vielzahl von Fähigkeitsprofilen und deren Zeitverlauf über die Behandlungsdauer bereitgestellt, die von einem historischen Patienten-Kollektiv stammen. Hierbei stehen zumindest die quantitativen, digitalen Daten des jeweiligen Patientenzustandes zu Beginn der Therapie sowie die Daten für den Verlauf der Therapie zur Verfügung. In einem ersten Schritt zur Bildung einer Vergleichskurve werden optional aus der Datenbank 2 der historischen Patientendaten diejenigen Vergleichs-Patienten ausgewählt, die mit dem aktuell behandelten Patienten nach vorher festgelegten Kriterien in ausreichendem Maße übereinstimmen. Die Kriterien für eine Übereinstimmung sind je nach Fähigkeitsprofil bzw. betrachtetem Krankheitsbild und Therapieplan unterschiedlich. Im vorliegenden Beispiel sind diese Kriterien in einer Wissensdatenbank 3 abgelegt, so dass sie auf Abruf zur Verfügung stehen. Diese Wissensdatenbank 3 enthält Ähnlichkeits- bzw. Ausschlusskriterien für die Auswahl von Patienten aus dem Patienten-Kollektiv, die vergleichbar mit dem gerade behandelten Patienten sind und der gleichen Therapie unterzogen werden. Weiterhin kann diese Datenbank Berechnungsvorschriften zur Berechnung einer Maßzahl bzw. eines Scores für die jeweilige Therapie enthalten, mit der bzw. dem der Therapiefortschritt quantifiziert werden kann. Durch die Bereitstellung der Auswahlkriterien in der Wissensdatenbank 3 kann die Selektion eines geeigneten Sub-Kollektivs von Vergleichs-Patienten automatisch durch eine Datenverarbeitungsstation vorgenommen werden. Kriterien bzw. Parameter für die Auswahl der historischen Patientendaten können beispielsweise die Mindestanzahl von übereinstimmenden Fähigkeitsdefiziten und die Maximalzahl von weiteren Fähigkeitsdefiziten bei den Vergleichs-Patienten sein, die beim gerade behandelten Patienten nicht auftreten. Weiterhin können als Kriterien eine Ober- und Untergrenze für die Abweichung des Grades des Defizits bei jeder Fähigkeit vom gerade behandelten Patienten angegeben werden. Weitere Kriterien sind ein ähnliches Alter, das gleiche Geschlecht oder gewisse weitere Erkrankungen als Ausschlusskriterium. Selbstverständlich ist diese Aufzählung nicht abschließend und hängt vom jeweiligen Zustandsprofil bzw. Krankheitsbild des zu therapierenden Patienten ab.

Nach diesem Schritt 4 der Auswahl eines Sub-Kollektivs aus den Vergleichs-Patienten des historischen Patienten-Kollektivs wird in Schritt 5 eine für ein Benchmarking geeignete Maßzahl berechnet. Diese für den Therapiefortschritt repräsentative Maßzahl wird wiederum vorzugsweise durch eine Berechnungsvorschrift aus den Messwerten des Fähigkeitsprofils erhalten, die durch die Wissensdatenbank 3 zur Verfügung gestellt wird. Für die Berechnung dieser Maßzahl werden alle für eine Therapie-Verlaufskontrolle geeigneten Messwerte mit Hilfe einer geeigneten mathematischen Formel, der Berechnungsvorschrift, verknüpft, um eine einzige Maßzahl, den Score des Therapieerfolgs, zu erhalten. Im einfachsten Falle kann beispielsweise nur der Mittelwert der Messwerte, d.h. der Mittelwert des Grades aller Defizite, berechnet und als Score bzw. Maßzahl verwendet werden. Selbstverständlich können jedoch auch andere mathematische Operationen, wie eine Multiplikation der Messwerte, ein geometrisches Mittel, eine gewichtete Mittelwertbildung usw., eingesetzt werden, die eine ähnliche zusammenfassende Funktion haben.

Wahlweise ist es auch möglich, nicht aus allen verfügbaren Messwerten für den Therapieverlauf einen Score zu berechnen, sondern einzelne Messwerte zu Untergruppen zusammenzufassen, um mehrere Maßzahlen bzw. Scores daraus zu berechnen. So kann beispielsweise der Therapiefortschritt bei allen Defiziten koordinativer Fähigkeiten anders und unabhängig von der Entwicklung aller kognitiver Fähigkeiten sein, so dass diese getrennt zusammengefasst und im Rahmen der Überwachung mit der Referenzgruppe verglichen werden müssen.

Im nächsten Schritt 6 wird aus den auf diese Weise berechneten Maßzahlen bzw. Scores des Sub-Kollektivs, die ein Maß für den Therapieerfolg bzw. Therapiefortschritt zu unterschiedlichen Zeitpunkten während der Therapie darstellen, eine Therapie-Verlaufskurve gebildet. Fehlende Maßzahlen zu bestimmten Zeitpunkten können dabei durch Interpolation zwischen den einzelnen Erfassungszeitpunkten erzeugt werden. Dieser Fall kann auftreten, wenn die Erhebung des Fähigkeitsprofils im Therapieverlauf nicht für alle Vergleichs-Patienten in den gleichen Zeitabständen durchgeführt wurde. Die Interpolation muss hierbei selbstverständlich bereits vor der Verknüpfung der Zahlen der einzelnen Vergleichspatienten, beispielsweise durch Mittelwertbildung, erfolgen.

Alternativ kann statt der Absolutwerte der Fähigkeitsdefizite oder anderer Werte für den Therapiefortschritt auch die Veränderung der Defizite gegenüber dem Ausgangspunkt der Behandlung berechnet werden. Eine derartige Angabe der Veränderung bzw. Verbesserung des Defizits ist für die Bewertung des Behandlungserfolges aussagekräftiger als ein erreichter Absolutwert.

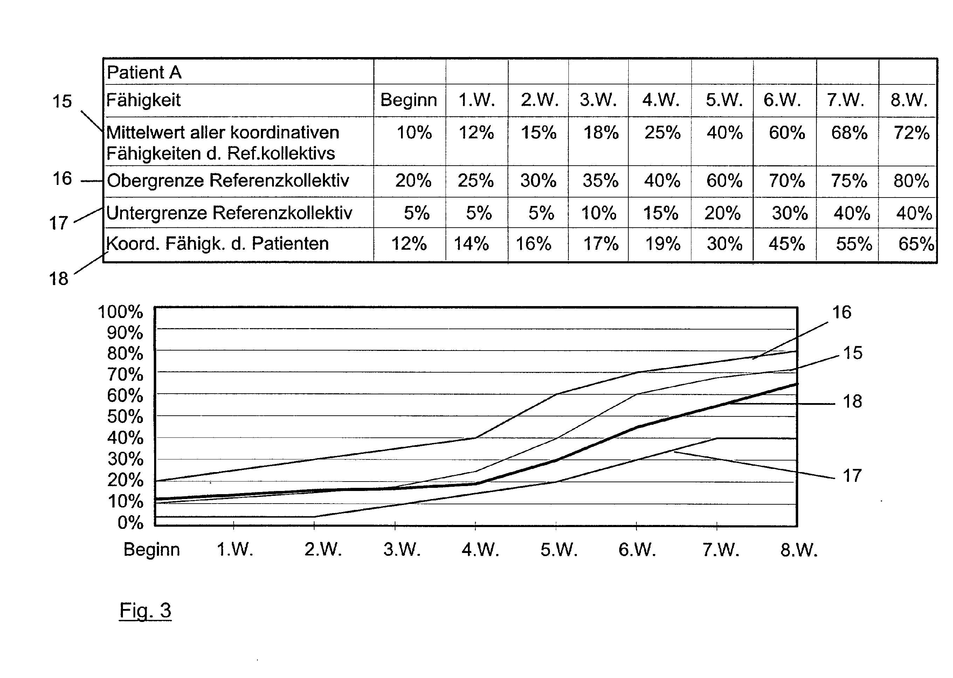

Nach der Bildung der Vergleichskurve aus dem historischen Sub-Kollektiv wird ein zulässiges Abstandsmaß bzw. eine zulässige Ober- und Untergrenze für die Abweichung der Maßzahl des Therapieverlaufs des gerade zu therapierenden Patienten von der Vergleichskurve festgelegt (Schritt 7). Für diese Festlegung können unterschiedliche Verfahren verwendet werden. So kann bei einer Mittelwertbildung zum Erhalt der Vergleichskurve die Standardabweichung der jeweils zu einzelnen Zeitpunkten berechneten Maßzahlen des Sub-Kollektivs herangezogen werden und daraus die zulässigen Grenzwerte, beispielsweise durch Verdopplung des Wertes der Standardabweichung, abgeleitet werden. Weiterhin ist es möglich, zwei Vergleichs-Patienten aus dem Sub-Kollektiv auszuwählen, die das beste Therapieergebnis und das schlechteste noch als Erfolg zu bezeichnende Therapieergebnis erzielt haben, und deren Verlaufskurven als Obergrenze 16 und Untergrenze 17 einzusetzen, wie dies in der später erläuterten Figur 3 vorgenommen wurde.

Um die Aussagekraft eines derartigen durch Ober- und Untergrenze begrenzten Referenzbandes zu stärken, kann statt des besten und des schlechtesten Falles die Ober- und Untergrenze beispielsweise durch den Mittelwert der besten bzw. schlechtesten 10% des Sub-Kollektivs berechnet werden.

Durch die vorangegangenen Verfahrensschritte steht somit eine Vergleichskurve bzw. Trendkurve über den erwarteten Verlauf des Trainingserfolges über die Behandlungsdauer hinweg zur Verfügung, die aus einem großen Patienten-Kollektiv errechnet wurde und als Benchmark mit dem Therapiefortschritt des gerade in Behandlung befindlichen Patienten verglichen werden kann.

Im Behandlungsverlauf wird dann zu verschiedenen Zeitpunkten oder in verschiedenen Therapiestufen das jeweils aktuelle individuelle Fähigkeitsprofil des Patienten gemessen und daraus mit der Berechnungsvorschrift die entsprechende repräsentative Maßzahl für den Therapiefortschritt berechnet (Schritt 8). Diese Maßzahl wird nun in Schritt 10 mit der Vergleichs-Kurve des Sub-Kollektivs verglichen, so dass zu jedem Zeitpunkt der Behandlung eine Aussage darüber möglich ist, ob sich dieser Patient im Vergleich zur Referenzgruppe des Sub-Kollektivs besser oder schlechter entwickelt. Auch der Unterschied zur Referenzgruppe, beispielsweise in Prozent der Defizite, ist quantifizierbar. Weiterhin kann auch in Schritt 9 aus den bisherigen Maßzahlen des Patienten eine Verlaufs-Kurve über die bisherige Therapiedauer gebildet werden und diese mit der Vergleichs-Kurve verglichen werden.

Fig. 3 zeigt ein Beispiel für eine aus einem Sub-Kollektiv berechnete Vergleichs-Kurve 15 über den Therapieverlauf, die einen Mittelwert aller koordinativen Fähigkeiten der Vergleichs-Patienten des Sub-Kollektivs darstellt. In der Figur ist ein Referenzband um diese Vergleichs-Kurve 15 zu erkennen, das durch eine obere Grenzkurve 16 und eine untere Grenzkurve 17 gebildet wird. Diese Grenzkurven entsprechen dem Therapieverlauf des Vergleichs-Patienten mit dem jeweils besten Therapieerfolg und des Vergleichs-Patienten mit dem jeweils schlechtesten Therapieerfolg innerhalb des gewählten Sub-Kollektivs. In dieser Darstellung ist auch der vollständige Therapieverlauf 18 des individuellen zu therapierenden Patienten dargestellt. Die Abweichung des Therapieverlaufs dieses individuellen Patienten von der Vergleichs-Kurve 15 ist deutlich zu erkennen. Der Therapiefortschritt des Patienten hinsichtlich seiner koordinativen Fähigkeiten bleibt jedoch zu jeder Zeit innerhalb des vorgegebenen Referenzbandes.

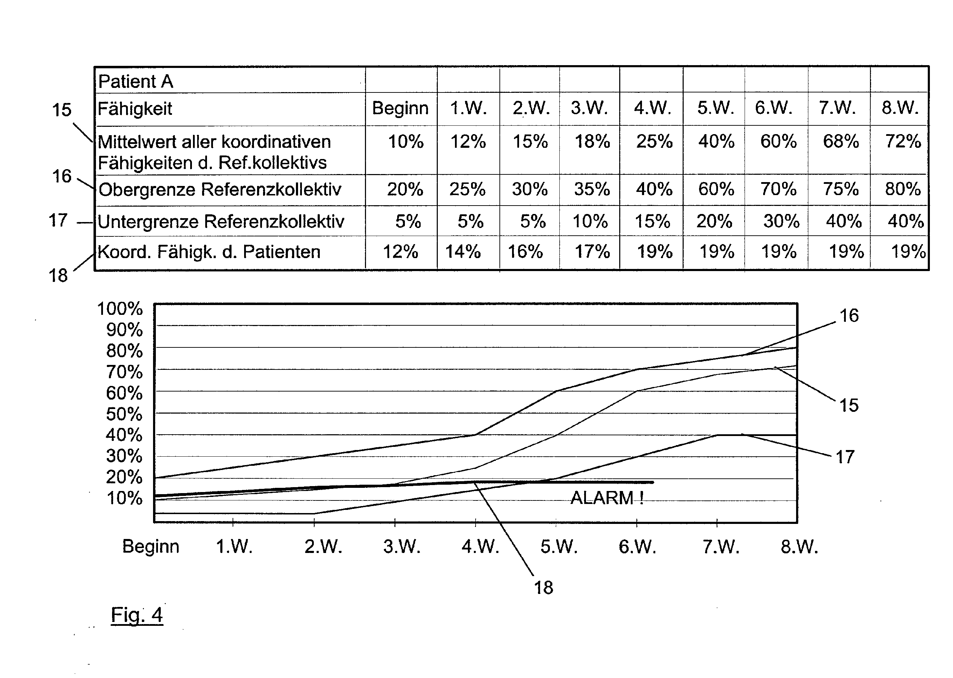

Zusätzlich kann auch ein Monitoring dahingehend stattfinden, ob der Patient aus dem Referenzband der Referenzgruppe herausfällt. Dies ist in Fig. 4 beispielhaft dargestellt. Auch in dieser Figur ist wiederum die Vergleichs-Kurve 15 mit dem Mittelwert aller koordinativen Fähigkeiten des Referenz-Kollektivs zu erkennen. Das Referenzband mit Obergrenze 16 und Untergrenze 17 wurde in gleicher Weise wie in Zusammenhang mit der Fig. 3 erläutert, gebildet. Bei diesem Beispiel fällt der Therapiefortschritt der koordinativen Fähigkeiten des Patienten, wie er durch die entsprechende Verlaufs-Kurve 18 in der Figur zu erkennen ist, am Ende der fünften Woche aus dem Referenzband heraus.

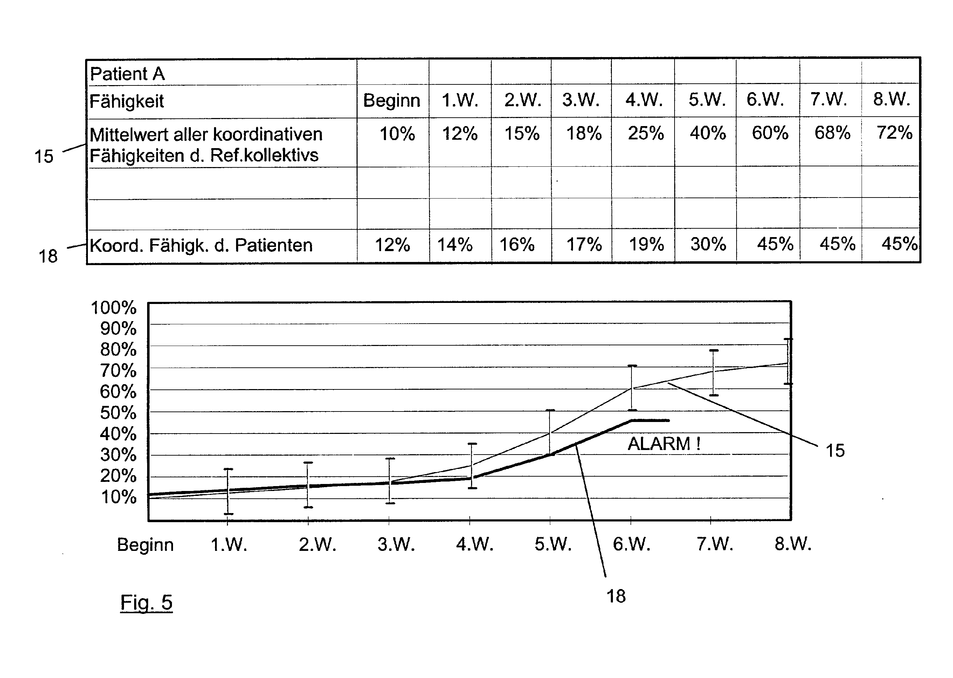

Bei diesem Unterschreiten des schlechtesten Vergleichsfalles kann eine Intervention erforderlich sein, um die Ursache dieses schlechten Ergebnisses zu klären und gegebenenfalls die Behandlungsstrategie zu ändern. Bei Überschreiten der oberen Grenze des Referenzbandes kann der Arzt sich entscheiden, entweder die Behandlung zu beenden oder bei weiterem Verbesserungspotential eine anspruchsvollere Therapieform zu wählen. In jedem Falle erfolgt im vorliegenden Beispiel eine Warn-, Alarm- oder Informationsmeldung an den behandelnden Therapeuten oder Arzt, sobald das Überschreiten des Referenzbandes erfasst wird (Schritte 11, 12). Selbstverständlich kann in diesem Zusammenhang auch die Überschreitung einer anderen Grenze, beispielsweise eines vorgegebenen Abstandes des Fähigkeitsprofils des Patienten zum Betrachtungszeitpunkt von der Vergleichs-Kurve, als auslösendes Ereignis für eine Warn-, Alarm- oder Informations-Meldung herangezogen werden. Dies ist beispielhaft durch die Darstellung in Fig. 5 veranschaulicht. In dieser Figur ist wiederum die Vergleichs-Kurve 15 mit dem Mittelwert aller koordinativen Fähigkeiten des Referenz-Kollektivs zu erkennen. Durch die auf dieser Vergleichs-Kurve 15 eingezeichneten Abstandsbalken wird wiederum ein Referenzband definiert, innerhalb dessen der Therapiefortschritt des individuellen Patienten, veranschaulicht durch die Therapie-Verlaufskurve 18, liegen muss. Auch hier wird beim Verlassen dieses Referenzbandes eine Alarmmeldung ausgelöst.

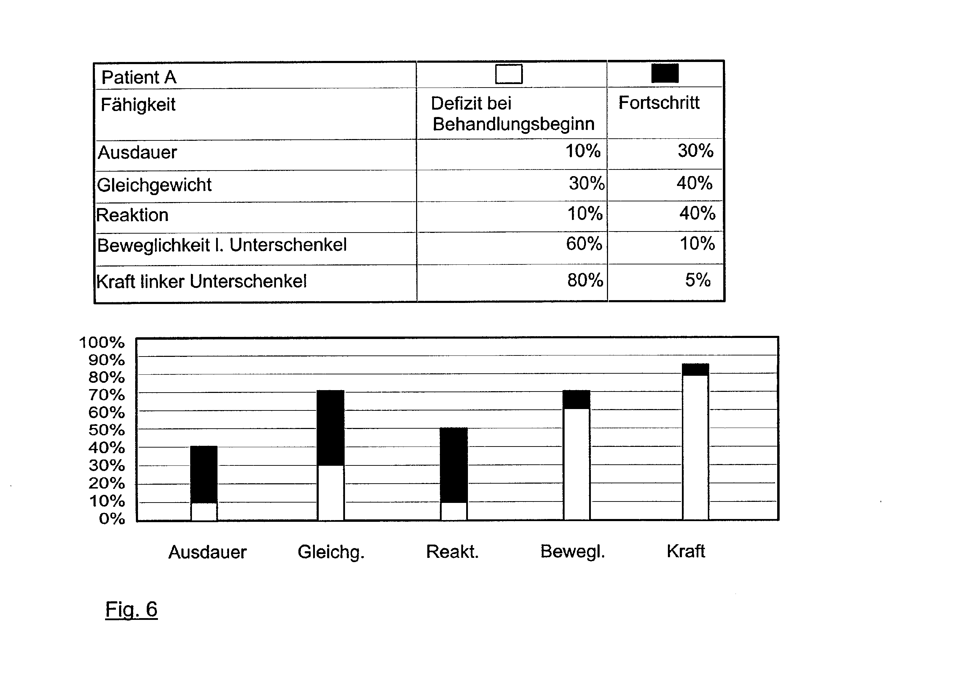

Neben der Ausgabe des Vergleichsergebnisses kann auch der momentane Therapiefortschritt, aufgegliedert nach den einzelnen Defiziten, in geeigneter Weise veranschaulicht werden, wie dies in der Fig. 6 dargestellt ist. Hier wird der Fortschritt bezüglich der Fähigkeiten, Ausdauer, Gleichgewicht, Reaktion, Beweglichkeit des linken Unterschenkels und Kraft im linken Unterschenkel bezogen auf den Behandlungsbeginn grafisch dargestellt. Der Arzt oder Therapeut kann aus dieser Darstellung sofort die Fähigkeiten erkennen, für die sich ein signifikanter Fortschritt ergeben hat und umgekehrt. Selbstverständlich werden in dieser Darstellung zum Vergleich in der Regel auch die diesem Zeitpunkt innerhalb der Therapie entsprechenden Daten der Vergleichskurve bzw. des Vergleichs-Kollektivs angezeigt.

Während bei diesem Ausführungsbeispiel als Bewertungsgröße für den Therapiefortschritt einer rehabilitativen Maßnahme die Fähigkeitsdefizite des Patienten herangezogen wurden, lassen sich selbstverständlich auch andere Zustandsprofile und Maßzahlen für den Therapiefortschritt in der Rehabilitation wählen. So können in analoger Weise neben dem Fähigkeitsprofil der Lebensqualitäts-Index, Ergebnisse von Computer-basierten Staging-Tests oder Ergebnisse der Übungsdurchführung herangezogen werden. Computer-basierte Staging-Tests haben den Vorteil, dass sie in vorteilhafter Weise an einem im häuslichen Umfeld des Patienten bereitgestellten Computer-Arbeitsplatz durchgeführt werden können und keinerlei externe Schnittstellen und Messwertaufnehmer erfordern.

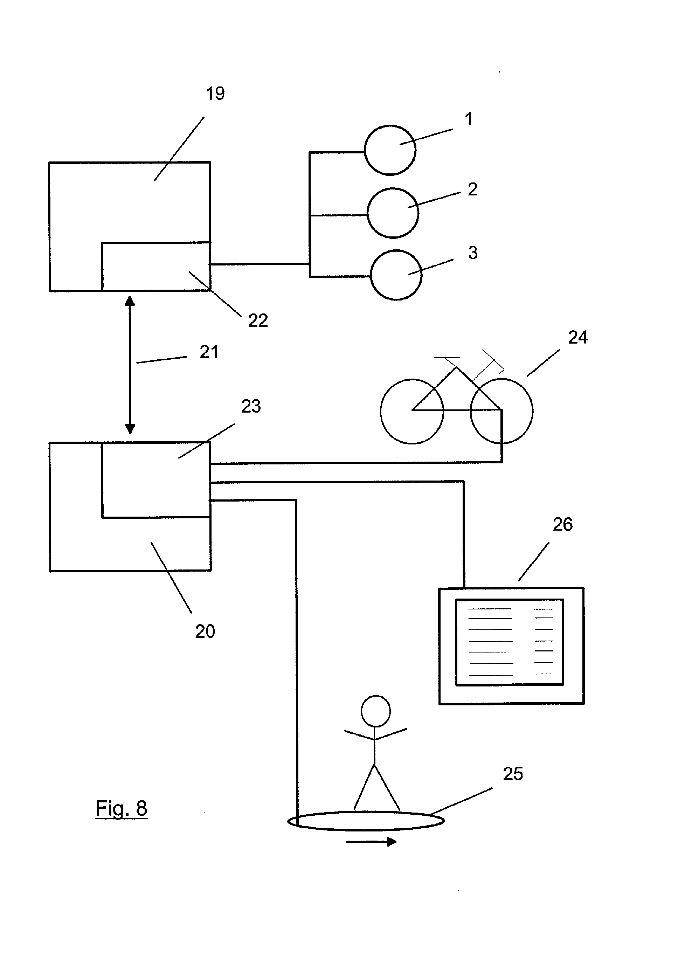

Fig. 8 zeigt beispielhaft und stark schematisiert den Aufbau eines Systems zur Durchführung des Verfahrens. Das System besteht aus einer ersten Datenverarbeitungsanlage 19 am Arbeitsplatz des Therapeuten oder Arztes sowie einer zweiten Datenverarbeitungsstation 20 im häuslichen Umfeld des zu therapierenden Patienten. Beide Datenverarbeitungsstationen sind zumindest zeitweise über ein Netzwerk 21 für den Datenaustausch miteinander verbunden. Die erste Datenverarbeitungsstation 19 verfügt über ein Modul 22, das mit den entsprechenden Datenbanken 1, 2 und 3 für den Abruf bzw. die Abspeicherung der für das Verfahren erforderlichen Daten verbunden ist. Das Programm-Modul 22 dient auch der Auswahl des Sub-Kollektivs aus den Vergleichs-Patienten, der Berechnung der entsprechenden Maßzahlen sowie der Bildung und Speicherung der Vergleichs-Kurve. Über dieses Modul 22 wird ferner der automatische Vergleich der jeweils aktuellen Maßzahl des Patienten mit der Vergleichs-Kurve durchgeführt und gegebenenfalls entsprechende Warn- oder Informations-Hinweise erzeugt. Die zweite Datenverarbeitungsstation 20 dient der Unterstützung des rehabilitativen Trainings des Patienten und ist gegebenenfalls mit Messwertaufnehmern an entsprechenden Trainingsgeräten 24, 25 verbunden. Bei der Messung kognitiver Fähigkeiten kann auch ein entsprechender Computer-gestützter Test bzw. ein Fragebogen an einem Monitor 26 zur Beantwortung dargestellt werden. Das in dieser zweiten Datenverarbeitungsstation 20 enthaltene Programm-Modul 23 dient dieser Messdaten-Erfassung sowie der Übermittlung der Messwerte oder bereits aus den Messwerten berechneter Maßzahlen an die erste Datenverarbeitungsstation 19 über das Netzwerk 21.

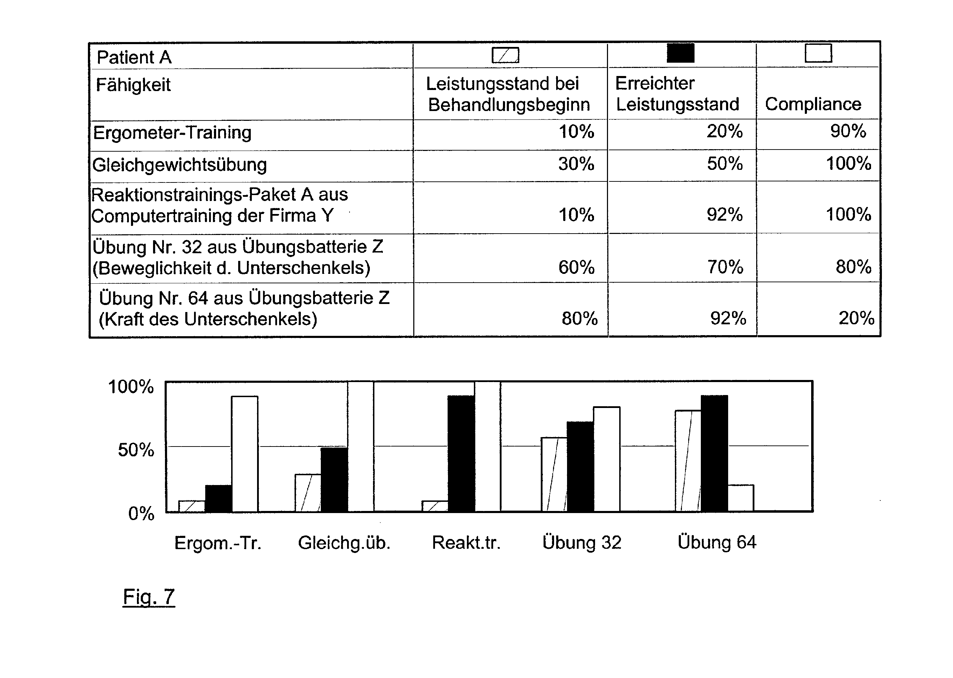

In einer weiteren Ausführungsform des Verfahrens kann das beschriebene Monitoring des Therapieverlaufs durch ein Monitoring der Compliance des Patienten ergänzt werden (Schritt 13). Diese Compliance, d.h. die Kooperationsbereitschaft und Kooperationsfähigkeit des Patienten, bei der Durchführung der Rehabilitationsmaßnahmen kann den Therapiefortschritt in erheblichem Ausmaß beeinflussen. Daher kann die Kenntnis über die Compliance des Patienten bei gleichem Stand des Therapieerfolgs zu unterschiedlichen Konsequenzen führen. Bei guter Compliance und stagnierendem Therapiefortschritt kann die Therapie eventuell beendet werden, da eine weitere Verbesserung nicht zu erwarten ist. Bei schlechter Compliance und stagnierendem Therapiefortschritt könnten Maßnahmen zur Verbesserung der Compliance eingeleitet werden. Bleibt die Compliance trotz Intervention schlecht und stagniert der Therapiefortschritt weiterhin, so kann eventuell die Therapie beendet oder die Kostenübernahme für die Therapie gestrichen werden. Fig. 7 zeigt ein Beispiel für eine Darstellung des zu einem bestimmten Zeitpunkt während der Therapie erreichten Leistungsstandes und der zugehörigen Compliance. Aus dieser Figur ist beispielsweise zu erkennen, dass sich die Reaktion des Patienten bei hervorragender Compliance erheblich verbessert hat, während die Verbesserung in der Kraft des Unterschenkels bei schlechter Compliance des Patienten nur geringfügig ausfällt.

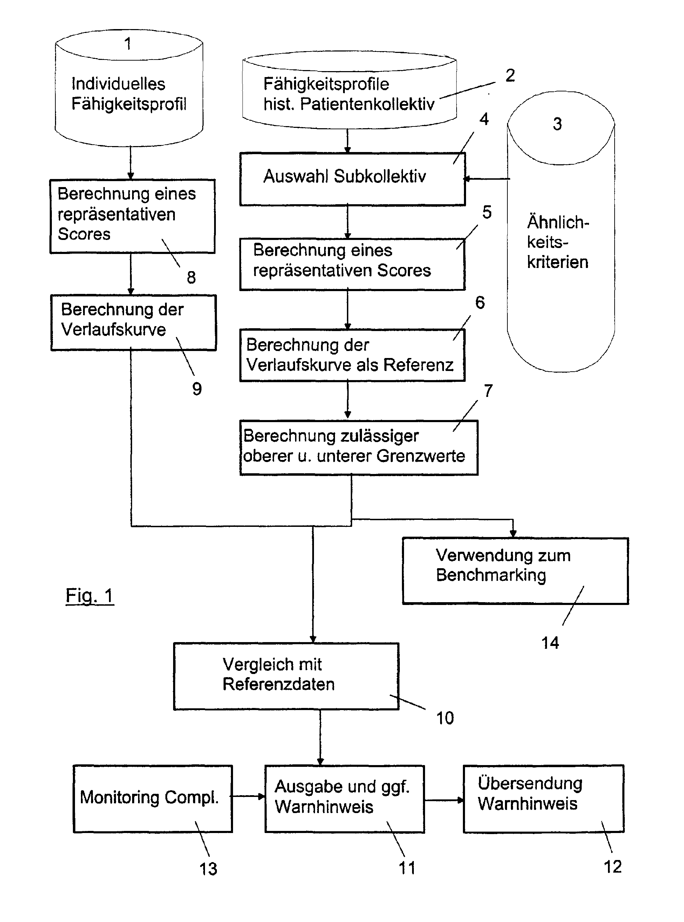

Einige der in der Fig. 1 dargestellten Verfahrensschritte bilden für sich allein ein Verfahren zur Bereitstellung von Vergleichsdaten für die Überwachung des Fortschritts oder die Bewertung des Erfolges einer Therapie. Dieses Verfahren kann neben der beschriebenen Beurteilung des Therapiefortschritts eines individuellen Patienten auch als Verfahren zum Benchmark der Qualität verschiedener Rehabilitations-Institutionen, beispielsweise zum internen Benchmarking innerhalb einer Institution verwendet werden (Schritt 14). In diesem Fall werden die Trend-Kurven der Patienten-Kollektive der zu vergleichenden Institutionen in der beschriebenen Art und Weise berechnet und die Kurven selbst oder daraus abgeleitete Maßzahlen verglichen. Derartige aus den Vergleichs-Kurven abgeleitete Maßzahlen können beispielsweise eine erreichte Qualitätsverbesserung als Mittelwert und Standardabweichung, die durchschnittliche Dauer bis zum Erreichen des vorgegebenen Therapiezieles oder eine erreichte Qualitätsverbesserung bezogen auf die aufgewendete Behandlungsdauer sein.
In diesem Fall muss selbstverständlich jeweils ein entsprechendes Krankheitsbild bzw. Zustandsprofil vorgegeben werden, auf das sich die Bereitstellung der Vergleichsdaten bezieht. Weiterhin kann auch die Berechnung der Vergleichskurve unterlassen werden, indem direkt aus den vorhandenen Patientendaten eine abschließende Maßzahl für die Bewertung des Therapieerfolges berechnet wird. Ein wesentlicher Bestandteil dieses gesonderten Verfahrens ist der Einsatz einer Wissensdatenbank 3 für die automatisierte Auswahl der entsprechenden Sub-Kollektive aus den zur Verfügung stehenden Patientendaten.

Obwohl das vorliegende Verfahren und System zur Überwachung des Therapieverlaufs eines Patienten gegen Erfahrungswerte bzw. Messwerte eines historischen Patienten-Kollektivs in der vorliegenden Beschreibung in erster Linie am Beispiel einer Rehabilitationsmaßnahme beschrieben ist, kann sich das Verfahren sowie das System selbstverständlich auch unmittelbar auf andere Therapiemaßnahmen übertragen, insbesondere auf die Therapie von chronischen Krankheiten wie Diabetes, Asthma, Herzinsuffizienz usw., welche zunehmend von so genannten Disease Management Services angeboten werden.

## Patentansprüche

1. Verfahren zur Überwachung des Therapieverlaufs einer medizinischen Behandlung eines Patienten
mit folgenden Schritten:
- Erstellen eines ersten Zustandsprofils des Patienten durch Messung eines oder mehrerer zu therapierender Defizite zu Beginn der Therapie, aus denen mit einer Berechnungsvorschrift eine für den Therapiefortschritt repräsentative Maßzahl berechnet wird;
- Bereitstellen von Therapieverlaufsdaten einer Vielzahl von zu einem Patientenkollektiv zusammengefassten Vergleichs-Patienten, die eine vergleichbare Therapie bereits durchgeführt haben;
- Bildung und Speicherung einer Therapieverlaufskurve aus den Therapieverlaufsdaten des Patienten-Kollektivs mit Hilfe der Berechnungsvorschrift für die Maßzahl als Vergleichskurve (15);
- Erstellen weiterer Zustandsprofile des Patienten durch Messung der zu therapierenden Defizite und Berechnen der jeweils aktuellen Maßzahl zu unterschiedlichen Zeitpunkten bzw. in unterschiedlichen Therapiestufen im Verlauf der Therapie; und
- Darstellen der jeweils aktuellen Maßzahl zusammen mit der dem jeweiligen Zeitpunkt bzw. der jeweiligen Therapiestufe entsprechenden Maßzahl der abgespeicherten Vergleichskurve (15) und/oder automatischer Vergleich der jeweils aktuellen Maßzahl mit der dem jeweiligen Zeitpunkt bzw. der jeweiligen Therapiestufe entsprechenden Maßzahl der abgespeicherten Vergleichskurve (15) und Ausgeben des Vergleichsergebnisses durch eine erste Datenverarbeitungsstation (19).

2. Verfahren nach Anspruch 1,
**dadurch gekennzeichnet,**
**dass** die Messung der zu therapierenden Defizite im Verlauf der Therapie automatisch durch eine zweite Datenverarbeitungsstation (20) erfolgt, die gegebenenfalls über eine oder mehrere Schnittstellen mit Messwertaufnehmern verbunden ist und die gemessenen Werte und/oder die daraus berechnete aktuelle Maßzahl über ein Netzwerk (21) an die erste Datenverarbeitungsstation (19) übermittelt.

3. Verfahren nach Anspruch 2,
**dadurch gekennzeichnet,**
**dass** die Therapie als telemedizinische Behandlung durchgeführt wird, wobei die zweite Datenverarbeitungsstation (20) im häuslichen Umfeld des Patienten steht.

4. Verfahren nach einem der Ansprüche 1 bis 3,
**dadurch gekennzeichnet,**
**dass** eine zulässige Abweichung der aktuellen Maßzahl von der Vergleichskurve (15) festgelegt und numerisch oder graphisch durch die erste Datenverarbeitungsstation (19) dargestellt wird.

5. Verfahren nach einem der Ansprüche 1 bis 4,
**dadurch gekennzeichnet,**
**dass** eine zulässige Abweichung der aktuellen Maßzahl von der Vergleichskurve (15) festgelegt wird und die erste (19) und/oder zweite Datenverarbeitungsstation (20) bei Überschreiten der Abweichung eine Warnmeldung erzeugt.

6. Verfahren nach einem der Ansprüche 1 bis 5,
**dadurch gekennzeichnet,**
**dass** das Bereitstellen von Therapieverlaufsdaten einer Vielzahl von Vergleichs-Patienten durch Zugriff auf eine erste Datenbank (2) erfolgt, die diese Therapieverlaufsdaten enthält.

7. Verfahren nach einem der Ansprüche 1 bis 6,
**dadurch gekennzeichnet,**
**dass** aus dem Patienten-Kollektiv der Vergleichs-Patienten ein Sub-Kollektiv ausgewählt wird, das nur Vergleichs-Patienten mit einem innerhalb vorgebbarer Grenzwerte mit dem ersten Zustandsprofil des Patienten übereinstimmenden Zustandsprofil zu Beginn der Therapie umfasst, und die Therapieverlaufskurve nur aus den Therapieverlaufsdaten des Sub-Kollektivs gebildet wird.

8. Verfahren nach Anspruch 7,
**dadurch gekennzeichnet,**
**dass** für die Auswahl des Sub-Kollektivs aus den Vergleichs-Patienten erforderliche Parameter und gegebenenfalls die Berechnungsvorschrift für die Berechnung der repräsentativen Maßzahl aus einer zweiten Datenbank (3) erhalten werden, in der für eine Vielzahl von Zustandsprofilen Parameter für eine repräsentative Auswahl eines Sub-Kollektivs sowie gegebenenfalls eine Berechnungsvorschrift für eine repräsentative Maßzahl angegeben werden, und die Auswahl des Sub-Kollektivs durch die erste Datenverarbeitungsstation (19) automatisiert mit den aus der zweiten Datenbank (3) erhaltenen Parametern erfolgt.

9. Verfahren nach Anspruch 8,
**dadurch gekennzeichnet,**
**dass** die Parameter die Grenzwerte sind, innerhalb der das erste Zustandsprofil des Patienten mit den Zustandsprofilen der Vergleichs-Patienten übereinstimmen muss.

10. Verfahren nach Anspruch 9,
**dadurch gekennzeichnet,**
**dass** die Grenzwerte eine Mindestanzahl an übereinstimmenden Defiziten angeben, eine Maximalanzahl an Defiziten, die zusätzlich zu den Defiziten des zu therapierenden Patienten bei den Vergleichs-Patienten des Sub-Kollektivs auftreten dürfen, sowie Ober- und Untergrenzen für eine Übereinstimmung des Grades des jeweiligen Defizits.

11. Verfahren nach einem der Ansprüche 7 bis 10,
**dadurch gekennzeichnet,**
**dass** für die Auswahl des Sub-Kollektivs weitere Eigenschaften des Patienten, insbesondere dessen Alter und weitere Krankheiten, berücksichtigt werden.

12. Verfahren nach einem der Ansprüche 1 bis 11,
**dadurch gekennzeichnet,**
**dass** die Bildung der Therapieverlaufskurve durch eine zusammenfassende mathematische Operation, insbesondere eine Mittelung, über die aus den Therapieverlaufsdaten der einzelnen Vergleichs-Patienten berechneten repräsentativen Maßzahlen erfolgt.

13. Verfahren nach einem der Ansprüche 1 bis 12,
**dadurch gekennzeichnet,**
**dass** der Therapieverlauf zusätzlich anhand zumindest eines zweiten Zustandsprofils, das zu einer anderen Gruppe von Fähigkeiten gehört wie das erste Zustandsprofil, in gleicher Weise wie anhand des ersten Zustandsprofils überwacht wird.

14. Verfahren nach einem der Ansprüche 1 bis 13,
**dadurch gekennzeichnet,**
**dass** die Therapie ein Training des Patienten im Rahmen einer Rehabilitation oder ein telemedizinisches Behandlungsverfahren für chronisch kranke Patienten ist.

15. Verfahren nach einem der Ansprüche 1 bis 14,
**dadurch gekennzeichnet,**
**dass** die Zustandsprofile Fähigkeitsdefizite, Fertigkeitsdefizite oder Maßzahlen für die Lebensqualität umfassen.

16. Verfahren nach einem der Ansprüche 1 bis 15,
**dadurch gekennzeichnet,**
**dass** die Maßzahlen eine Veränderung der ein oder mehreren zu therapierenden Defizite gegenüber einem zu Beginn der Therapie bestehenden Anfangszustand angeben.

17. Verfahren nach einem der Ansprüche 1 bis 16,
**dadurch gekennzeichnet,**
**dass** dem Erfassungszeitpunkt der jeweils aktuellen Maßzahl entsprechende Maßzahlen der Vergleichskurve aus den Therapieverlaufsdaten interpoliert werden, falls für diesen Zeitpunkt keine Daten vorliegen.

18. Verfahren nach einem der Ansprüche 1 bis 17,
**dadurch gekennzeichnet,**
**dass** zusätzlich die Compliance des Patienten zu dem jeweiligen Zeitpunkt bzw. in der jeweiligen Therapiestufe erfasst und zusammen mit dem Vergleichsergebnis ausgegeben wird.

19. Verfahren zur Bereitstellung von Vergleichsdaten für die Überwachung des Fortschritts oder die Bewertung des Erfolges einer Therapie, mit folgenden Schritten:
- Vorgeben eines ersten Zustandsprofils eines Patienten, das ein oder mehrere zu therapierende Defizite umfasst;
- Bereitstellen von Therapieverlaufsdaten einer Vielzahl von Patienten durch eine erste Datenbank (2);
- Abrufen oder Vorgeben einer Berechnungsvorschrift für die Berechnung einer Maßzahl zur Bewertung des Therapiefortschrittes oder Therapieerfolges sowie Abrufen von Auswahlparametern für eine Auswahl eines repräsentativen Sub-Kollektivs aus der Vielzahl von Patienten aus einer zweiten Datenbank (3), in der für eine Vielzahl von Zustandsprofilen Parameter für eine repräsentative Auswahl eines Sub-Kollektivs sowie gegebenenfalls Berechnungsvorschriften für Maßzahlen zur Bewertung des Therapiefortschrittes oder Therapieerfolges angegeben werden;
- automatische Auswahl des Sub-Kollektivs durch eine erste Datenverarbeitungsstation (19) mit den aus der zweiten Datenbank (3) erhaltenen Parametern; und
- Berechnen einer oder mehrerer Maßzahlen zur Bewertung des Therapiefortschrittes oder Therapieerfolges aus den Therapieverlaufsdaten des Sub-Kollektivs mit der Berechnungsvorschrift durch die erste Datenverarbeitungsstation (19).

20. Verfahren nach Anspruch 19 zum Vergleich der Therapieerfolge unterschiedlicher medizinischer Leistungserbringer.

21. System zur Überwachung des Therapieverlaufs einer medizinischen Behandlung eines Patienten mit zumindest einer ersten Datenverarbeitungsstation (19) sowie einer zweiten Datenverarbeitungsstation (20), die zumindest zeitweise über ein Netzwerk (21) miteinander Daten austauschen können,
wobei die erste Datenverarbeitungsstation (19) mit einer ersten Datenbank (2) verbunden ist, die Therapieverlaufsdaten einer Vielzahl von zu einem Patienten-Kollektiv zusammengefassten Vergleichs-Patienten enthält, und ein erstes Modul (22) zur Bildung und Speicherung einer Therapie-Verlaufskurve (15) aus den Therapieverlaufsdaten des Patienten-Kollektivs mit Hilfe einer Berechnungsvorschrift für eine den Therapiefortschritt repräsentierenden Maßzahl, zur Berechnung der jeweils aktuellen Maßzahl des Patienten aus von der zweiten Datenverarbeitungsstation (20) übermittelten Messwerten sowie zur Darstellung der jeweils aktuellen Maßzahl zusammen mit der dem jeweiligen Zeitpunkt bzw. der jeweiligen Therapiestufe entsprechenden Maßzahl der abgespeicherten Vergleichskurve und/oder zum automatischen Vergleich der jeweils aktuellen Maßzahl mit der Therapie-Vergleichskurve (15) und zur Ausgabe des Vergleichsergebnisses umfasst, und
wobei die zweite Datenverarbeitungsstation (20) ein zweites Modul (23) zur Erfassung der Messwerte für die Berechnung der Maßzahl und zur Übertragung dieser Messwerte an die erste Datenverarbeitungsstation (19) umfasst.

22. System nach Anspruch 21,
**dadurch gekennzeichnet,**
**dass** die erste Datenverarbeitungsstation (9) mit einer zweiten Datenbank (3) verbunden ist, in der für eine Vielzahl von Zustandsprofilen Parameter für eine repräsentative Auswahl eines Sub-Kollektivs aus dem Patienten-Kollektiv der Vergleichs-Patienten sowie gegebenenfalls die Berechnungsvorschrift für die repräsentative Maßzahl angegeben sind, und das erste Modul (22) zur Auswahl des Sub-Kollektivs aus dem Patienten-Kollektiv auf Basis der von der zweiten Datenbank (3) erhaltenen Parameter und zur Bildung der Therapie-Verlaufskurve (15) aus den Therapieverlaufsdaten des Sub-Kollektivs ausgebildet ist.

23. System nach Anspruch 21 oder 22,
**dadurch gekennzeichnet,**
**dass** die zweite Datenverarbeitungsstation (20) über eine oder mehrere Schnittstellen mit Messwertaufnehmern zur Erfassung der Messwerte verbunden ist.
